# EUROPEAN PATENT SPECIFICATION

(11) **EP 2 368 491 B1**
(45) Date of publication and mention of the grant of the patent: **14.08.2019**
(21) Application number: 11158714.3
(22) Date of filing: 17.03.2011
(51) Int. Cl.: G16H 50/30

(54) **Method and device for determining an indicator of generator clinical state**
Verfahren und Vorrichtung zur Bestimmung eines Indikators des klinischen Generatorzustands
Procédé et dispositif permettant de déterminer un indicateur de l'état clinique d'un générateur

(30) Priority: 22.03.2010 US 728657
(43) Date of publication of application: 28.09.2011
(73) Proprietor: General Electric Company, Schenectady, NY 12345 (US)
(72) Inventor: Rantala, Borje Tor, 00670, Helsinki (FI); Palmer, Michael J., New Berlin, WI 53151 (US); Soosalu, Milvi, 02270, Espoo (FI); Luosta, Jutta, 01230, Vantaa (FI); Halinene, Henriikka, 00130, Helsinki (FI); Lahtevonoja, Paivi Hannele, 02610, Espoo (FI)
(74) Representative: Fennell, Gareth Charles

(56) References cited:
- WO-A1-2008/085309
- WO-A2-2004/059551
- US-A- 5 355 893
- US-A1- 2004 147 982
- US-A1- 2008 269 625
- US-A1- 2010 030 293

## Description

### Background of the Invention

This disclosure relates generally to patient monitoring. More particularly, the present invention relates to determination of an indicator that signifies the general clinical state of a patient in terms of planned care and that may be used as a guide in evaluating when the patient is clinically ready for or in need of planned care measures.

Patient monitors are electronic devices designed to display physiological information about a subject. Electrocardiogram (ECG), electroencephalogram (EEG), plethysmographic signals, and signals related to blood pressure, temperature, and respiration represent typical physiological information contained in full-size patient monitors. Patient monitors are typically also furnished with alarming functionality to alert the nursing staff when a vital sign or physiological parameter of a patient exceeds or drops below a preset limit. Alarms are normally both audible and visual effects aiming to alert the staff to a life-threatening condition or to another event considered vital. In most monitors, the alarm limits may be defined by the user, since the limits typically depend on patient etiology, age, gender, medication, and various other subjective factors. Each specific physiological parameter, such as heart rate or blood pressure, may also be assigned more than one alarm limit.

In addition to individual sensor/parameter alarms, patient monitors can be configured to raise combinatory alarms. That is, several physiological parameters may be used to determine a combined index and to give an alarm when the combined index fulfills a specific criterion. The combinatory alarms may range from simple combinations like "low heart rate and low arterial pressure" to complex rule-based scenarios used in various clinical support systems, for example.

WO 2008/085309 discloses a patient device comprising a module adapted to detect an alert status of each of one or more sensors, an analysis module adapted to calculate an alert score by combining the detected alerts and calculate a composite alert score indicative of a physiological condition.

One drawback of the use of the combinatory indices/alarms is that the more physiological parameters are used to determine the combinatory index, the more difficult it is for a clinician to grasp the connection between the behavior of the index and the underlying physiological behavior of the patient. Along with the increasing number of parameters, the determination rule of the combinatory index becomes more complex, especially if different weights are used for different parameters, i.e. components of the combinatory index. The alarm limits related to such indices typically signify specific events and it is important that the clinician is fully aware of the physiological meaning of the alarm for each patient, otherwise the use of the combinatory alarms leads to low appreciation of the features of the monitoring system.

Another drawback related to the present patient monitor systems is that both the individual sensor alarms and the combinatory indices are designed to watch over critical events that are more or less sudden in nature. Consequently, the present patient monitor systems can detect such events but cannot reflect the more general, albeit small and/or slow deterioration or improvement in the general clinical condition of the patient. Particularly, the present patient monitor systems fail to provide illustrative indicators for assisting the staff in deciding when the general clinical state of the patient has improved enough for the next phase of the planned care. The present patient monitor systems therefore cannot support the staff in deciding when the patient is ready for a new phase of therapy. Furthermore, clinical decision support systems are complex and expensive, due to the embedded diagnostic intelligence, and it would be desirable if common patient monitors with only modest computing power, and thus also modest cost, could be provided with an ability to track the slow and/or small changes in the general clinical state of the patient in view of the planned care.

### Brief Description of the Invention

The present invention is defined in the accompanying claims.

The above-mentioned problems are addressed herein which will be comprehended from the following specification.

In an embodiment, a method for determining a clinical index indicative of a general clinical state of a subject in terms of planned care includes selecting a set of physiological parameters for a type of care to be applied to a subject, the set of physiological parameter being determined from the subject. The method further includes attaching a targeted value range to each physiological parameter belonging to the set of physiological parameters and determining a general condition index of the subject as a function of at least two integers belonging to a group including (i) the number of physiological parameters of the set that are currently within respective targeted value ranges, (ii) the number of physiological parameters of the set that are currently outside respective targeted value ranges, and (iii) the total number of physiological parameters in the set of physiological parameters.

In another embodiment, an apparatus for determining a clinical index indicative of a general clinical state of a subject in terms of planned care includes a selection unit configured to select a set of physiological parameters for a type of care to be applied to a subject, the set of physiological parameter being determined from the subject. The apparatus further includes a range definition unit configured to associate a targeted value range with each physiological parameter belonging to the set of physiological parameters and a determination unit configured to determine a general condition index of the subject as a function of at least two integers belonging to a group including (i) the number of physiological parameters of the set that are currently within respective targeted value ranges, (ii) the number of physiological parameters of the set that are currently outside respective targeted value ranges, and (iii) the total number of physiological parameters in the set of physiological parameters.

In a still further embodiment, a computer program product for determining a clinical index indicative of a general clinical state of a subject in terms of planned care comprises a first program product portion configured to select a set of physiological parameters for a type of care to be applied to a subject, the set of physiological parameter being determined from the subject. The computer program product further comprises a second program product portion configured to associate a targeted value range with each physiological parameter belonging to the set of physiological parameters and a third program product portion configured to determine a general condition index of the subject as a function of two integers belonging to a group including (i) the number of physiological parameters of the set that are currently within respective targeted value ranges, (ii) the number of physiological parameters of the set that are currently outside respective targeted value ranges, and (iii) the total number of physiological parameters in the set of physiological parameters.

Various other features, objects, and advantages of the invention will be made apparent to those skilled in the art from the following detailed description and accompanying drawings. The invention is defined by the appended claims, the description serving for illustrative purpose.

### Brief Description of the Drawings

FIG. 1 illustrates one embodiment of an apparatus or system for monitoring a subject;
FIG. 2 is a flow diagram illustrating one embodiment of the operation of the apparatus of FIG. 1 for determining a general condition index of the subject;
FIG. 3 illustrates one embodiment of the initial user interaction phase of FIG. 2;
FIG. 4 is a flow diagram illustrating one embodiment of the index determination step;
FIG. 5 illustrates the operational entities of the control and processing unit of FIG. 1 in terms of the determination of the general condition index; and
FIG. 6 illustrates an example of a screen page displayed to the user for illustrating the general clinical condition of the patient in terms of the planned care.

### Detailed Description of the Invention

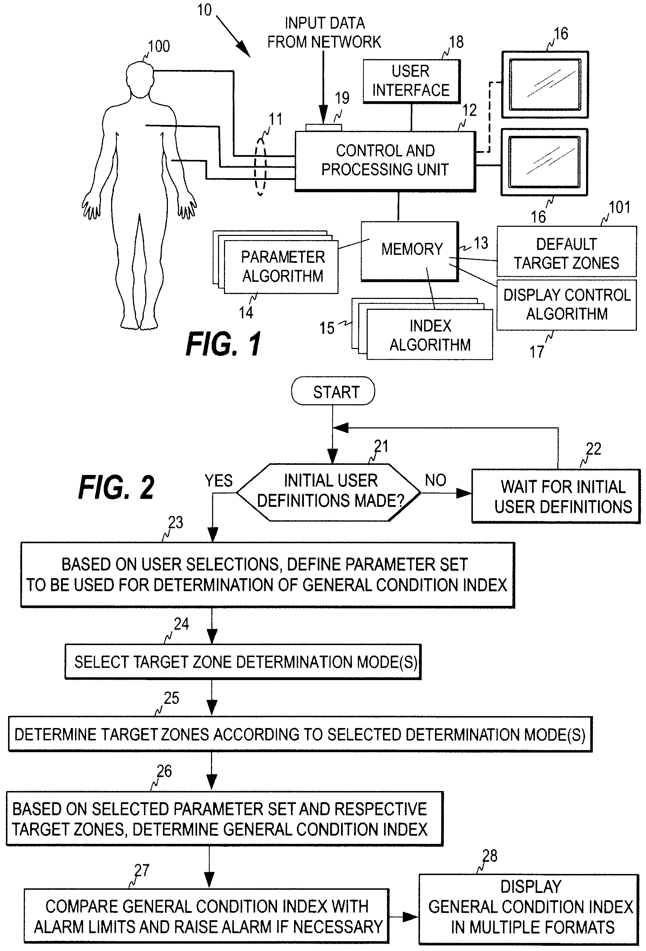

FIG. 1 illustrates one embodiment of a monitoring apparatus/system 10 for monitoring a subject 100. A monitoring apparatus/system normally acquires a plurality of physiological signals 11 from the subject, where one physiological signal corresponds to one measurement channel. The physiological signals typically comprise several types of signals, such as ECG, EEG, blood pressure, respiration, and plethysmographic signals. Based on the raw real-time physiological signal data obtained from the subject, a plurality of physiological parameters may be determined. A physiological parameter here refers to a variable calculated from the waveform data of one or more of the physiological signals acquired from the subject. If a physiological parameter is derived from more than one physiological signal, i.e. from more than one measurement channel, the said physiological signals are usually of the same signal type. The physiological parameter may thus also represent a waveform signal value determined over a predefined period of time, although the physiological parameter is typically a distinct parameter derived from one or more measurement channels, such as heart rate derived from an ECG signal or an SPO2 value derived from a plethysmographic signal. Each signal parameter may be assigned one or more alarm limits to alert the nursing staff when the parameter reaches or crosses the alarm limit.

The physiological signals 11 acquired from the subject 100 are supplied to a control and processing unit 12 through a pre-processing stage (not shown) comprising typically an input amplifier and a filter, for example. The control and processing unit converts the signals into digitized format for each measurement channel. The digitized signal data may then be stored in the memory 13 of the control and processing unit. The digitized signal data may be utilized by parameter algorithms 14 adapted to record, when executed by the control and processing unit, the time series of the physiological parameters. The obtained time series of the physiological parameters may be stored in the memory.

For the determination of the general condition index, the control and processing unit is provided with one or more index algorithms 15, each index algorithm being configured to determine a general condition index that reflects the general clinical condition of the subject in view of the type of care related to the algorithm. It is to be noted here that depending on the planned care the general clinical condition is evaluated from different points of view and therefore different physiological parameters are needed depending on the type of care. Below, ventilator weaning is used as an example of the type of care applied to the subject.

The control and processing unit is further configured to control the display unit 16 of the apparatus. A display control algorithm 17 may be stored in the memory of the control and processing unit and the apparatus may be provided with more than one display unit. The user may supply information and control the apparatus/system through user interface 18. Various input information, such as patient data, may also be input through a network interface 19. Further, all the physiological parameters are not necessarily determined by the control and processing unit based on the physiological waveform signals 11 measured from the subject at the bedside, but one or more of the physiological parameters needed in the determination of the general condition index may also be received through the network interface from a laboratory, for example.

FIG. 2 is a flow diagram illustrating one embodiment of the operation of the control and processing unit for determining the general condition index of the subject. First, an initial user interaction phase is carried out, in which the user may supply/select the initial data needed for the determination of the general condition index of the subject (steps 21 and 22). This may be carried out by selecting desired options through the user interface 18 of the apparatus. The control and processing unit then selects a set of physiological parameters to be used for the determination of the general condition index (step 23). The selection is made, based on the user-defined initial information, from among the physiological parameters defined from the subject.

Each physiological parameter may be provided with a default targeted value range that defines the range of values that the parameter should have normally. The targeted value range is termed target zone in this context. The default limits of the target zones may be stored in memory 13, as is denoted with reference number 101 in FIG. 1. Different mechanisms may then be used to adapt the target zones to the subject in question. Consequently, the apparatus may next select the target zone determination mode, i.e. the mechanism according to which the final limits of the target zones are determined, at step 24. After this, the target zones are determined according to the selected mode. The said mode may or may not be user-assisted. In step 24, the apparatus may examine, for example, whether the default limits of the target zones may be used for the subject in question. If this is not the case, the apparatus may choose to calculate subject-specific limit values based on patient information or give the user of the apparatus an opportunity to adjust the limits of the target zones from their default values. Consequently, in step 25 the control and processing unit may simply select default target zones, calculate subject-specific limits for the target zones based on patient information, or prompt the user to adjust the limits of the target zones of the selected physiological parameters from the default values stored in the apparatus. Different selection modes may also be used for different physiological parameters of the set, thereby to obtain the final target zone for each parameter of the set. For example, the control and processing unit may decide, based on patient information, that for one or more parameters default target zones may be used, while for the other parameters calculated and/or user-defined target zones are needed.

After the determination of the target zones, the control and processing unit determines the general condition index (step 26) based on the selected set of physiological parameters and the respective target zones, compares the obtained value of the index with preset alarm limit(s), and raises an alarm if necessary (step 27). The control and processing unit further displays the index in multiple formats to the user (step 28).

As the physiological waveform signals 11 are typically processed in fixed length time windows termed epochs, new values for physiological parameters are obtained at regular intervals, such as every 30 seconds. Steps 26 to 28 may be carried out accordingly, thereby to obtain a new index value at regular intervals, such as every 30 seconds.

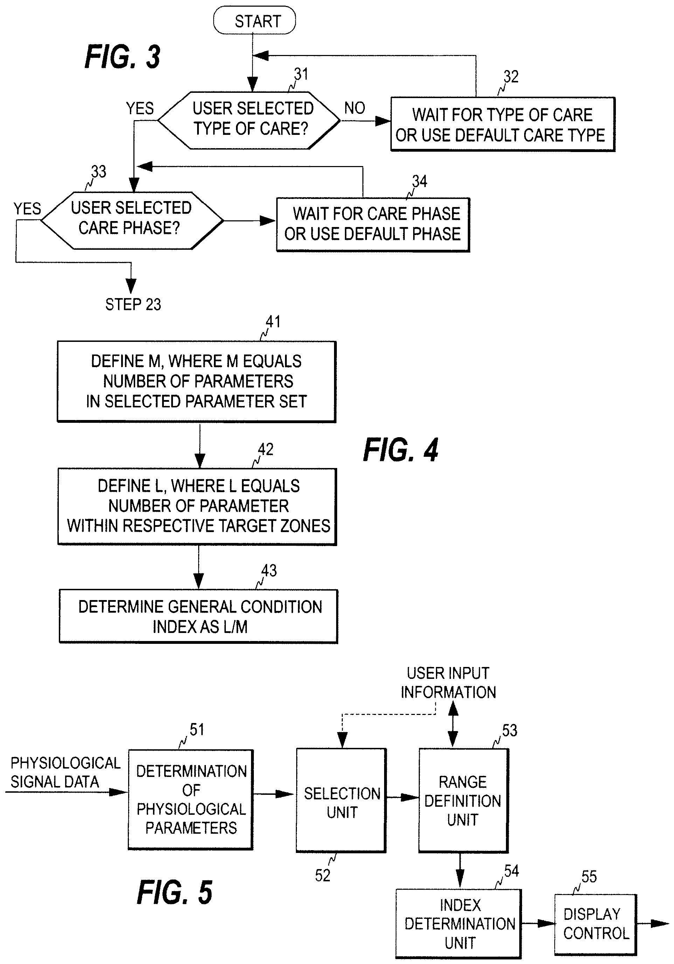

FIG. 3 illustrates an example of the initial user interaction phase (steps 21 and 22 of FIG.2). It is assumed here that the apparatus/system may be utilized for more than one type of care and therefore the user may first be given a chance to select the type of care to be applied to the subject (steps 31 and 32). Each care type may be associated with the respective index algorithm and thus the respective algorithm is selected in response to the selection of the type of care. In practice, the user may select the desired type of care from the care type menu of the apparatus. When the type of care has been selected in steps 31 and 32, the user may further be given a chance to select (step 33) the care phase to be applied to the subject. In ventilator weaning, for example, the phases prior to and after the disconnection of the subject from the ventilator set quite different requirements for the patient monitoring and therefore also the determination of the general condition index is different for the two care phases. When the user has defined the type and phase of care, the process jumps to step 23 to define the set of physiological parameters to be used for the determination of the general condition index. Depending on the type of care, the selection of the set may or may not require the definition of the care phase. Default values may also be selected by the apparatus for the care type and phase, as is shown in FIG. 3.

FIG. 4 illustrates an example of the determination of the general condition index in step 26. The control and processing unit first defines the number of parameters M (M≥2) in the parameter set selected in step 23. Using the target zones defined in steps 24 and 25, the control and processing unit then determines (step 42) the number of parameters L that are currently within the respective target zone. The general condition index is then determined as L/M, i.e. as the ratio of L to M. In this example, the index thus describes the proportion of parameters that are currently within the expected value range.

In step 27, the value of the ratio is compared with the alarm limit(s) of the index and an alarm is produced if necessary. It is to be noted that the individual parameters used for the index may each have their own alarm limit(s) and an alarm may be produced for each parameter individually.

In terms of the determination of the general condition index of the subject, the control and processing unit 12 of FIG. 1 may thus be seen as an entity of five operational modules or units, as is illustrated in FIG. 5: a parameter determination unit 51 configured to determine the time series of the physiological parameters for the measurement channels, a selection unit 52 configured to select the set of physiological parameters to be used for the index determination, a range definition unit 53 configured to attach a target zone to each parameter of the set, an index determination unit 54 configured to generate the general condition index based on the set of physiological parameters and based on the respective target zones, and a display control unit 55 configured to visualize the general condition index to the user. The display control unit may drive multiple display units. The index determination unit may comprise several index algorithms for defining the general condition index for several types of care. The range definition unit may retrieve the default target zone limits from the memory and adjust the limits according to user preferences.

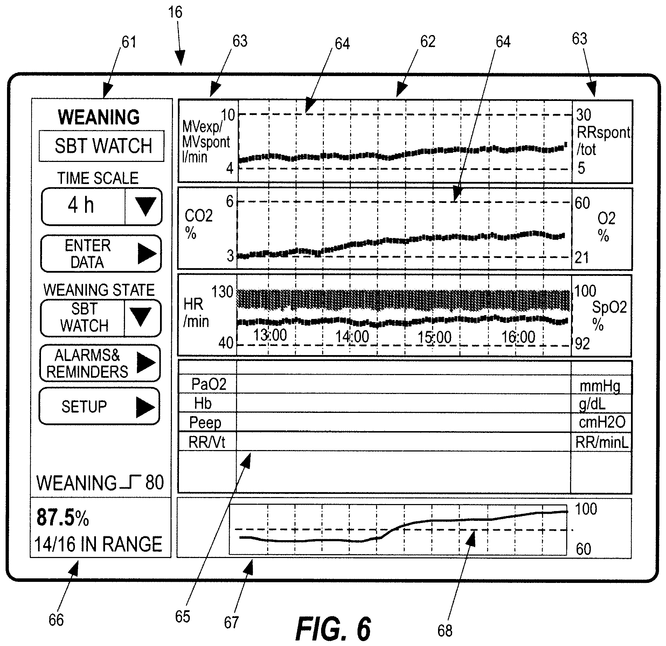

FIG. 6 illustrates example of a screen page displayed to the user in step 28. The screen page comprises a menu field 61 and several vertically aligned trend fields 62, each comprising two scales; left scale and right scale. At the right and left ends of each trend field, a parameter field 63 indicates the particular parameter of the respective scale. In each trend field, the trends of two physiological parameters may therefore be presented. In FIG. 6, the lowermost trend field shows the trends of two physiological parameters: SpO2 on the right scale and heart rate (HR) on the left scale. The SpO2 value is shown as a bar extending downwards from the value of 100. Each trend field further shows the target zones 64 of the respective parameter(s). In the figure, the target zones are shown by dashed lines, but in an actual patient monitor the area of the target zone may be coloured with a desired colour, such as green. The upper and lower limits of the target zones may be at fixed levels of the trend field. For example, the lower limit may be at a height corresponding to 10% and the upper limit at a height corresponding to 90% of the height of the trend field. The height of the target zone then corresponds to 80% of the total height of the trend field. Consequently, the height of the target zone remains fixed and the parameter values of the target zone limits are changed if the target zone is adjusted. For example, if the user wishes to adjust the HR limits 40 and 130 shown in the figure, the graphical area of the target zone is not adjusted but only the new limit values are shown on the HR scale and the scale is dimensioned according to the new limits.

The menu field of FIG. 6 indicates the type of care selected by the user in step 31, which is weaning in this example, and the care phase selected by the user in step 33, which is spontaneous breathing trial (SBT) in this example. The screen page of FIG. 6 further includes an information field 65 comprising horizontal rows, each relating to a further physiological parameter whose values are shown as a series of discrete values on the respective row. For these numerical trends, the target zone may usually not be visible directly, but color coding may be used to inform the user whether the value is within or outside the target zone. The parameters of the information field also typically belong to the parameter set selected in step 23, such as the parameters of the trend fields. However, the parameters of the information field may be imported or manually entered parameters, whereas the parameters of the trend fields are more typically determined by the control and processing unit.

The screen page of FIG. 6 further includes an index field 66 in which the current value of the general condition index may be presented as a ratio and as a percentage value, as is shown in the figure. The ratio also indicates the current values of L and M. The screen page may further include an index trend field 67 in which the index trend may be shown, together with the respective alarm threshold 68. The threshold value is 80% in this example, and the trend indicates that the threshold value has been exceeded about at 14:30 hrs. In this example, the crossing of the threshold suggests that the general clinical condition of the subject is good enough for a spontaneous breathing trial.

Depending on the value of M, the trends and the target zones of the selected physiological parameters may be presented on one or several screen pages and the general condition index may be presented on one or more of these pages. Furthermore, various mechanisms may be utilized to enhance the clarity of the presentation. For example, legends may be used to indicate which curves belong to the left scale and which to the right scale. The parameter or index trend graphs may also be displayed with different colors depending on the magnitude of the index or parameter value with respect to respective limit values.

A conventional patient monitor may be upgraded to enable the monitor to determine and display the general condition index. Such an upgrade may be implemented, for example, by delivering to the monitor a software module that may involve different functionality depending on the parameters available in the monitor. The software module may be delivered, for example, on a data carrier, such as a CD or a memory card, or the through a telecommunications network. Since the software module may utilize the physiological parameters already determined by the monitor, the module does not necessarily comprise more than three portions: a first program product portion configured to select a set of physiological parameters for the type of care to be applied to the subject, a second program product portion configured to associate a targeted value range with each physiological parameter belonging to the set of physiological parameters, and a third program product portion configured to determine a general condition index of the subject as a function of two integers belonging to a group including the number of physiological parameters of the set that are currently within respective targeted value ranges, the number of physiological parameters of the set that are currently outside respective targeted value ranges, and the total number of physiological parameters in the set of physiological parameters. However, the software module may also determine one or more of the physiological parameters, especially if all physiological parameters are not available in the monitor, and the module may be provided with a display control portion for the features of the index visualization. The determined parameters are thus typically new, derived parameters, such the ratio PaO2/FIO2. Regardless of whether or not the software of the control and processing unit is upgradable, the control and processing unit may also utilize physiological parameters transferred from an external entity, such as a laboratory or an external data system. The set of physiological parameters selected for a type of care to be applied to the subject may thus include internally determined parameters and/or parameters imported from an external entity. The apparatus may also be implemented as an auxiliary apparatus/unit connectable to an existing patient monitor. In this embodiment, the apparatus/unit may comprise the functionality of the software module, for example.

The general condition index provides a single global descriptor for the condition of the subject in terms of the type of care applied to the subject. Although the descriptor is a combinatory index, it is clear for clinician how the index is formed and how the components affect the final index value. This global descriptor may also be adapted to the subject in question by selecting the parameters based on which the index is determined and/or by adjusting the limits of the targeted value range of one or more parameter. The "weight" of a single parameter may be adjusted by extending or narrowing the target zone, so that it will be more likely that the parameter value will be within the target zone. Yet, the "weight" of a parameter remains clear to the user, since the limits of the target zone can be seen from the screen. It is therefore also easy for the user to modify the effect of a physiological parameter on the index.

In step 24, the user may also be given a chance to adjust the set of parameters, not only the target zones. The general condition index may also be defined as the ratio (M-L)/M, where M-L represents the number of physiological parameters that are currently outside the respective target zones. This may be used, for example, to track if the general condition of the subject is deteriorating. Furthermore, the general condition index may also be determined as the ratio L/(M-L), (M-L)/L, M/L, or M/(M-L). All ratios reflect the balance between parameters that are and are not within their expected value ranges. Thus, an alert can be specified as high or low limit violation of "in range" or "out of range" percentage.

Instead of the ratio of the two integers, another function of two or three integers selected or derived from among L, M-L, and M may be used, such as a difference, a ratio of differences, or a square of a ratio. The integer 2L-M, for example, directly indicates whether a majority of the parameters are within the respective target zones. The result obtained may further be scaled or normalized to a desired index scale.

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural or operational elements that do not differ from the literal language of the claims, or if they have structural or operational elements with insubstantial differences from the literal language of the claims.

## Claims

1. A method for determining a clinical index indicative of a general clinical state of a subject in terms of planned care, the method comprising:
- selecting (23), using a control and processing unit (12), a set of physiological parameters for a type of care to be applied to a subject (100), the set of physiological parameter being determined from the subject;
- acquiring a plurality of physiological signals (11) from the subject (100);
- attaching (25), using a control and processing unit (12), a targeted value range (64) to each physiological parameter belonging to the set of physiological parameters; and
- determining (26), using a control and processing unit (12), a general condition index (66) of the subject as a ratio of at least two integers belonging to a group including (i) the number of physiological parameters of the set that are currently within respective targeted value ranges, (ii) the number of physiological parameters of the set that are currently outside respective targeted value ranges, and (iii) the total number of physiological parameters in the set of physiological parameters.

2. The method according to claim 1, wherein the determining includes
- determining the general condition index as the ratio of the number of physiological parameters of the set that are currently within respective targeted value ranges to the total number of physiological parameters in the set, or
- determining the general condition index as the ratio of the number of physiological parameters of the set that are currently outside respective targeted value ranges to the total number of physiological parameters in the set.

3. The method according to claim 1 or claim 2, further comprising displaying (28) the general condition index in multiple formats.

4. The method according to any preceding claim, wherein the attaching includes retrieving a default targeted value range (101) for each physiological parameter belonging to the set of physiological parameters and prompting a user to adjust the default targeted value range of at least one of the physiological parameters of the set, thereby to obtain the targeted value range for each physiological parameter belonging to the set of physiological parameters.

5. The method according to any preceding claim, further comprising
- acquiring physiological signals from the subject; and
- defining a plurality of physiological parameters based on the physiological signals, wherein the selecting includes selecting the set of physiological parameters from the plurality of physiological parameters.

6. The method according to any preceding claim, further comprising displaying current values of at least some of the physiological parameters of the set, wherein the displaying includes using color coding to indicate whether the current values are within respective targeted value ranges.

7. An apparatus for determining a clinical index indicative of a general clinical state of a subject in terms of planned care, the apparatus comprising:
- a selection unit (52) configured to select a set of physiological parameters for a type of care to be applied to a subject (100), the set of physiological parameter being determined from the subject;
- a range definition unit (53) configured to associate a targeted value range (64) with each physiological parameter belonging to the set of physiological parameters; and
- a determination unit (54) configured to determine a general condition index of the subject as a ratio of at least two integers belonging to a group including (i) the number of physiological parameters of the set that are currently within respective targeted value ranges, (ii) the number of physiological parameters of the set that are currently outside respective targeted value ranges, and (iii) the total number of physiological parameters in the set of physiological parameters.

8. The apparatus according to claim 7, wherein the determination unit (54) is configured to determine the general condition index (1) as the ratio of the number of physiological parameters of the set that are currently within respective targeted value ranges to the total number of physiological parameters in the set or (2) as the ratio of the number of physiological parameters of the set that are currently outside respective targeted value ranges to the total number of physiological parameters in the set.

9. The apparatus according to claim 7 or claim 8, further comprising a display unit (16) configured to display the general condition index in multiple formats.

10. The apparatus according to any of claims 7 to 9, wherein the range definition unit (53) is configured to retrieve a default targeted value range for each physiological parameter belonging to the set of physiological parameters and to prompt a user to adjust the default targeted value range of at least one physiological parameter of the set, thereby to obtain the targeted value range for each physiological parameter belonging to the set of physiological parameters.

11. The apparatus according to any of claims 7 to 10, further comprising a parameter determination unit (51) configured to acquire physiological signals from the subject and to define a plurality of physiological parameters based on the physiological signals, wherein the selection unit is configured to select the set of physiological parameters from the plurality of physiological parameters.

12. The apparatus according to any of claims 7 to 11, wherein the display unit (16) is further configured to display current values of at least some of the physiological parameters of the set, and wherein the display unit is further configured to use color coding to indicate whether the current values are within respective targeted value ranges.

13. A computer program product for determining a clinical index indicative of a general clinical state of a subject in terms of planned care, the computer program product comprising:
- a first program product portion configured to select a set of physiological parameters for a type of care to be applied to a subject (100), the set of physiological parameter being determined from the subject;
- a second program product portion configured to associate a targeted value range (64) with each physiological parameter belonging to the set of physiological parameters; and
- a third program product portion configured to determine a general condition index of the subject as a ratio of two integers belonging to a group including (i) the number of physiological parameters of the set that are currently within respective targeted value ranges, (ii) the number of physiological parameters of the set that are currently outside respective targeted value ranges, and (iii) the total number of physiological parameters in the set of physiological parameters.

## Patentansprüche

1. Verfahren zur Bestimmung eines klinischen Index, der für den klinischen Allgemeinzustand eines Subjekts in Hinblick auf eine geplante Versorgung indikativ ist, wobei das Verfahren umfasst:
- unter Verwendung einer Steuer- und Verarbeitungseinheit (12) Auswählen (23) eines Satzes von physiologischen Parametern für einen Typ von Versorgung, der für ein Subjekt (100) angewendet werden soll, wobei der Satz von physiologischen Parametern an dem Subjekt bestimmt wird;
- Aufnehmen einer Vielzahl von physiologischen Signalen (11) von dem Subjekt (100);
- unter Verwendung einer Steuer- und Verarbeitungseinheit (12) Zuordnen (25) eines Sollwertbereichs (64) zu jedem physiologischen Parameter, der zu dem Satz von physiologischen Parametern gehört; und
- unter Verwendung einer Steuer- und Verarbeitungseinheit (12) Bestimmen (26) eines Allgemeinzustand-Index (66) des Subjekts als das Verhältnis von wenigstens zwei ganzen Zahlen, die zu einer Gruppe gehören, die (i) die Zahl von physiologischen Parametern des Satzes, die aktuell innerhalb entsprechender Sollwertbereiche liegen, (ii) die Zahl von physiologischen Parametern des Satzes, die aktuell außerhalb entsprechender Sollwertbereiche liegen, und (iii) die Gesamtzahl von physiologischen Parametern in dem Satz von physiologischen Parametern umfasst.

2. Verfahren gemäß Anspruch 1, wobei das Bestimmen umfasst:
- Bestimmen des Allgemeinzustand-Index als das Verhältnis der Zahl von physiologischen Parametern des Satzes, die aktuell innerhalb entsprechender Sollwertbereiche liegen, zu der Gesamtzahl von physiologischen Parametern in dem Satz oder
- Bestimmen des Allgemeinzustand-Index als das Verhältnis der Zahl von physiologischen Parametern des Satzes, die aktuell außerhalb entsprechender Sollwertbereiche liegen, zu der Gesamtzahl von physiologischen Parametern in dem Satz.

3. Verfahren gemäß Anspruch 1 oder Anspruch 2, ferner umfassend Anzeigen (28) des Allgemeinzustand-Index in verschiedenen Formaten.

4. Verfahren gemäß einem der vorstehenden Ansprüche, wobei das Zuordnen Abrufen eines vorgegebenen Sollwertbereichs (101) für jeden physiologischen Parameter, der zu dem Satz von physiologischen Parametern gehört, und Auffordern eines Anwenders, den vorgegebenen Sollwertbereich wenigstens eines der physiologischen Parameter in dem Satz abzugleichen, um den Sollwertbereich für jeden physiologischen Parameter, der zu dem Satz von physiologischen Parametern gehört, zu erhalten.

5. Verfahren gemäß einem der vorstehenden Ansprüche, ferner umfassend:
- Aufnehmen von physiologischen Signalen von dem Subjekt; und
- Definieren einer Vielzahl von physiologischen Parametern auf der Grundlage der physiologischen Signale, wobei das Auswählen das Auswählen des Satzes von physiologischen Parametern aus der Vielzahl von physiologischen Parametern umfasst.

6. Verfahren gemäß einem der vorstehenden Ansprüche, ferner umfassend Anzeigen von aktuellen Werten wenigstens mancher der physiologischen Parameter des Satzes, wobei das Anzeigen die Verwendung von Farbcodierung umfasst, um anzuzeigen, ob die aktuellen Werte innerhalb entsprechender Sollwertbereiche liegen.

7. Vorrichtung zur Bestimmung eines klinischen Index, der für den klinischen Allgemeinzustand eines Subjekts in Hinblick auf eine geplante Versorgung indikativ ist, wobei die Vorrichtung umfasst:
- eine Auswahleinheit (52), gestaltet zum Auswählen eines Satzes von physiologischen Parametern für einen Typ von Versorgung, der für ein Subjekt (100) angewendet werden soll, wobei der Satz von physiologischen Parametern an dem Subjekt bestimmt wird;
- eine Bereichsdefinitionseinheit (53), gestaltet zum Zuordnen eines Sollwertbereichs (64) zu jedem physiologischen Parameter, der zu dem Satz von physiologischen Parametern gehört; und
- eine Bestimmungseinheit (54), gestaltet zum Bestimmen eines Allgemeinzustand-Index des Subjekts als das Verhältnis von wenigstens zwei ganzen Zahlen, die zu einer Gruppe gehören, die (i) die Zahl von physiologischen Parametern des Satzes, die aktuell innerhalb entsprechender Sollwertbereiche liegen, (ii) die Zahl von physiologischen Parametern des Satzes, die aktuell außerhalb entsprechender Sollwertbereiche liegen, und (iii) die Gesamtzahl von physiologischen Parametern in dem Satz von physiologischen Parametern umfasst.

8. Vorrichtung gemäß Anspruch 7, wobei die Bestimmungseinheit (54) dafür gestaltet ist, den Allgemeinzustand-Index (1) als das Verhältnis der Zahl von physiologischen Parametern des Satzes, die aktuell innerhalb entsprechender Sollwertbereiche liegen, zu der Gesamtzahl von physiologischen Parametern in dem Satz oder (2) als das Verhältnis der Zahl von physiologischen Parametern des Satzes, die aktuell außerhalb entsprechender Sollwertbereiche liegen, zu der Gesamtzahl von physiologischen Parametern in dem Satz zu bestimmen.

9. Vorrichtung gemäß Anspruch 7 oder Anspruch 8, ferner umfassend eine Anzeigeeinheit (16), die zum Anzeigen des Allgemeinzustand-Index in verschiedenen Formaten gestaltet ist.

10. Vorrichtung gemäß einem der Ansprüche 7 bis 9, wobei die Bereichsdefinitionseinheit (53) dafür gestaltet ist, einen vorgegebenen Sollwertbereich für jeden physiologischen Parameter, der zu dem Satz von physiologischen Parametern gehört, abzurufen und einen Anwenders aufzufordern, den vorgegebenen Sollwertbereich wenigstens eines der physiologischen Parameter in dem Satz abzugleichen, um den Sollwertbereich für jeden physiologischen Parameter, der zu dem Satz von physiologischen Parametern gehört, zu erhalten

11. Vorrichtung gemäß einem der Ansprüche 7 bis 10, ferner umfassend eine Parameterbestimmungseinheit (51), die dafür gestaltet ist, physiologische Signale von dem Subjekt aufzunehmen und eine Vielzahl von physiologischen Parametern auf der Grundlage der physiologischen Signale zu definieren, wobei die Auswahleinheit dafür gestaltet ist, den Satz von physiologischen Parametern aus der Vielzahl von physiologischen Parametern auszuwählen.

12. Vorrichtung gemäß einem der Ansprüche 7 bis 11, wobei die Anzeigeeinheit (16) ferner dafür gestaltet ist, aktuelle Werte wenigstens mancher der physiologischen Parameter des Satzes anzuzeigen, und wobei die Anzeigeeinheit ferner dafür gestaltet ist, eine Farbcodierung zu verwenden, um anzuzeigen, ob die aktuellen Werte innerhalb entsprechender Sollwertbereiche liegen.

13. Computerprogrammprodukt zur Bestimmung eines klinischen Index, der für den klinischen Allgemeinzustand eines Subjekts in Hinblick auf eine geplante Versorgung indikativ ist, wobei das Computerprogrammprodukt umfasst:
- einen ersten Programmproduktteil, gestaltet zum Auswählen eines Satzes von physiologischen Parametern für einen Typ von Versorgung, der für ein Subjekt (100) angewendet werden soll, wobei der Satz von physiologischen Parametern an dem Subjekt bestimmt wird;
- einen zweiten Programmproduktteil, gestaltet zum Zuordnen eines Sollwertbereichs (64) zu jedem physiologischen Parameter, der zu dem Satz von physiologischen Parametern gehört; und
- einen dritten Programmproduktteil, gestaltet zum Bestimmen eines Allgemeinzustand-Index des Subjekts als das Verhältnis von wenigstens zwei ganzen Zahlen, die zu einer Gruppe gehören, die (i) die Zahl von physiologischen Parametern des Satzes, die aktuell innerhalb entsprechender Sollwertbereiche liegen, (ii) die Zahl von physiologischen Parametern des Satzes, die aktuell außerhalb entsprechender Sollwertbereiche liegen, und (iii) die Gesamtzahl von physiologischen Parametern in dem Satz von physiologischen Parametern umfasst.

## Revendications

1. Procédé pour déterminer un indice clinique indiquant un état clinique général d'un sujet en termes de soins planifiés, le procédé consistant :
- à sélectionner (23), à l'aide d'une unité de commande et de traitement (12), un ensemble de paramètres physiologiques pour un type de soins à appliquer à un sujet (100), l'ensemble de paramètres physiologiques étant déterminé à partir du sujet ;
- à acquérir une pluralité de signaux physiologiques (11) à partir du sujet (100) ;
- à attribuer (25), à l'aide d'une unité de commande et de traitement (12), une plage de valeurs ciblées (64) à chaque paramètre physiologique appartenant à l'ensemble de paramètres physiologiques ; et
- à déterminer (26), à l'aide d'une unité de commande et de traitement (12), un indice d'état général (66) du sujet comme étant un rapport d'au moins deux nombres entiers appartenant à un groupe comprenant (i) le nombre de paramètres physiologiques de l'ensemble qui se trouvent à présent à l'intérieur de plages de valeurs ciblées respectives, (ii) le nombre de paramètres physiologiques de l'ensemble qui se trouvent à présent à l'extérieur de plages de valeurs ciblées respectives et (iii) le nombre total de paramètres physiologiques dans l'ensemble de paramètres physiologiques.

2. Procédé selon la revendication 1, dans lequel la détermination consiste :
- à déterminer l'indice d'état général comme étant le rapport entre le nombre de paramètres physiologiques de l'ensemble qui se trouvent à présent à l'intérieur de plages de valeurs ciblées respectives et le nombre total de paramètres physiologiques dans l'ensemble ou
- à déterminer l'indice d'état général comme étant le rapport entre le nombre de paramètres physiologiques de l'ensemble qui se trouvent à présent à l'extérieur de plages de valeurs ciblées respectives et le nombre total de paramètres physiologiques dans l'ensemble.

3. Procédé selon la revendication 1 ou la revendication 2, consistant en outre à afficher (28) l'indice d'état général dans de multiples formats.

4. Procédé selon l'une quelconque des revendications précédentes, dans lequel l'attribution consiste à récupérer une plage de valeurs ciblées par défaut (101) pour chaque paramètre physiologique appartenant à l'ensemble de paramètres physiologiques et à inviter un utilisateur à ajuster la plage de valeurs ciblées par défaut d'au moins l'un des paramètres physiologiques de l'ensemble, pour obtenir ainsi la plage de valeurs ciblées pour chaque paramètre physiologique appartenant à l'ensemble de paramètres physiologiques.

5. Procédé selon l'une quelconque des revendications précédentes, consistant en outre :
- à acquérir des signaux physiologiques à partir du sujet ; et
- à définir une pluralité de paramètres physiologiques en se basant sur les signaux physiologiques, dans lequel la sélection consiste à sélectionner l'ensemble de paramètres physiologiques à partir de la pluralité de paramètres physiologiques.

6. Procédé selon l'une quelconque des revendications précédentes, consistant en outre à afficher des valeurs actuelles d'au moins quelques-uns des paramètres physiologiques de l'ensemble, dans lequel l'affichage consiste à utiliser un codage en couleurs pour indiquer si les valeurs actuelles se trouvent à l'intérieur de plages de valeurs ciblées respectives.

7. Appareil pour déterminer un indice clinique indiquant un état clinique général d'un sujet en termes de soins planifiés, l'appareil comprenant :
- une unité de sélection (52) configurée pour sélectionner un ensemble de paramètres physiologiques pour un type de soins à appliquer à un sujet (100), l'ensemble de paramètres physiologiques étant déterminé à partir du sujet ;
- une unité de définition de plage (53) configurée pour associer une plage de valeurs ciblées (64) à chaque paramètre physiologique appartenant à l'ensemble de paramètres physiologiques ; et
- une unité de détermination (54) configurée pour déterminer un indice d'état général du sujet comme étant un rapport d'au moins deux nombres entiers appartenant à un groupe comprenant (i) le nombre de paramètres physiologiques de l'ensemble qui se trouvent à présent à l'intérieur de plages de valeurs ciblées respectives, (ii) le nombre de paramètres physiologiques de l'ensemble qui se trouvent à présent à l'extérieur de plages de valeurs ciblées respectives et (iii) le nombre total de paramètres physiologiques dans l'ensemble de paramètres physiologiques.

8. Appareil selon la revendication 7, dans lequel l'unité de détermination (54) est configurée pour déterminer l'indice d'état général (1) comme étant le rapport entre le nombre de paramètres physiologiques de l'ensemble qui se trouvent à présent à l'intérieur de plages de valeurs ciblées respectives et le nombre total de paramètres physiologiques dans l'ensemble ou (2) comme étant le rapport entre le nombre de paramètres physiologiques de l'ensemble qui se trouvent à présent à l'extérieur de plages de valeurs ciblées respectives et le nombre total de paramètres physiologiques dans l'ensemble.

9. Appareil selon la revendication 7 ou la revendication 8, comprenant en outre une unité d'affichage (16) configurée pour afficher l'indice d'état général dans de multiples formats.

10. Appareil selon l'une quelconque des revendications 7 à 9, dans lequel l'unité de définition de plage (53) est configurée pour récupérer une plage de valeurs ciblées par défaut pour chaque paramètre physiologique appartenant à l'ensemble de paramètres physiologiques et pour inviter un utilisateur à ajuster la plage de valeurs ciblées par défaut d'au moins un paramètre physiologique de l'ensemble, pour obtenir ainsi la plage de valeurs ciblées pour chaque paramètre physiologique appartenant à l'ensemble de paramètres physiologiques.

11. Appareil selon l'une quelconque des revendications 7 à 10, comprenant en outre une unité de détermination de paramètre (51) configurée pour acquérir des signaux physiologiques à partir du sujet et pour définir une pluralité de paramètres physiologiques en se basant sur les signaux physiologiques, dans lequel l'unité de sélection est configurée pour sélectionner l'ensemble de paramètres physiologiques à partir de la pluralité de paramètres physiologiques.

12. Appareil selon l'une quelconque des revendications 7 à 11, dans lequel l'unité d'affichage (16) est en outre configurée pour afficher des valeurs actuelles d'au moins quelques-uns des paramètres physiologiques de l'ensemble, et dans lequel l'unité d'affichage est en outre configurée pour utiliser un codage en couleurs pour indiquer si les valeurs actuelles se trouvent à l'intérieur de plages de valeurs ciblées respectives.

13. Produit-programme d'ordinateur pour déterminer un indice clinique indiquant un état clinique général d'un sujet en termes de soins planifiés, le produit-programme d'ordinateur comprenant :
- une première partie de produit-programme configurée pour sélectionner un ensemble de paramètres physiologiques pour un type de soins à appliquer à un sujet (100), l'ensemble de paramètres physiologiques étant déterminé à partir du sujet ;
- une deuxième partie de produit-programme configurée pour associer une plage de valeurs ciblées (64) à chaque paramètre physiologique appartenant à l'ensemble de paramètres physiologiques ; et
- une troisième partie de produit-programme configurée pour déterminer un indice d'état général du sujet comme étant un rapport de deux nombres entiers appartenant à un groupe comprenant (i) le nombre de paramètres physiologiques de l'ensemble qui se trouvent à présent à l'intérieur de plages de valeurs ciblées respectives, (ii) le nombre de paramètres physiologiques de l'ensemble qui se trouvent à présent à l'extérieur de plages de valeurs ciblées respectives et (iii) le nombre total de paramètres physiologiques dans l'ensemble de paramètres physiologiques.
